# EUROPEAN PATENT SPECIFICATION

(11) **EP 2 638 179 B1**
(45) Date of publication and mention of the grant of the patent: **26.11.2014**
(21) Application number: 11775913.4
(22) Date of filing: 25.10.2011
(51) Int. Cl.: C14C 3/26, C07D 239/30, C07D 251/44

(54) **NON METAL TANNING**
GERBUNG OHNE METALLVERBINDUNGEN MIT GERBWIRKUNG
TANNAGE SANS COMPOSÉS MÉTALLIQUES À L'ACTIVITÉ TANNAGE

(30) Priority: 12.11.2010 EP 10014550
(43) Date of publication of application: 18.09.2013
(73) Proprietor: Stahl International B.V., 5145 PE Waalwijk (NL)
(72) Inventor: REINEKING, Claus, 71111 Waldenbuch (DE); GAMARINO, Roberta, I-15033 Casale Monferrato (IT); TRIMARCO, Licia, I-21047 Saronno (VA) (IT); QUAGLIERINI, Maurizio, 56034 Chianni ( PI) (IT); GISLER, Markus, CH-4310 Rheinfelden (CH); NUSSER, Rainer, 79395 Neuenburg (DE)
(74) Representative: Jansen, Cornelis Marinus
(86) International application number: PCT/EP2011/005367
(87) International publication number: WO 2012/062413

(56) References cited:
- WO-A1-94/10345
- GB-A- 838 337
- US-A- 3 068 190
- BOWLES J H ET AL: "Crosslinking of Collagen", JOURNAL OF APPLIED CHEMISTRY, SOCIETY OF CHEMICAL INDUSTRY. LONDON, GB, vol. 15, 15 July 1965 (1965-07-15), pages 296-304, XP002549304, cited in the application

## Description

In the production of leather, the tanning process is of decisive importance for the properties and quality of the resulting leather. Among the various tannings known in the art, i.e. mineral, vegetable and synthetic, chrome-based tannage is a conventional and widely spread way of tanning which is readily feasible in an industrial scale on most various kinds of skins and hides, and provides tanned leathers with satisfactory properties (such as high shrinkage temperatures T_{S}, suppleness and suitability for subsequent processing such as neutralization, retanning, fat-liquoring, dyeing, finishing).

Chrome-based tanning agents and tannings are however considered environmentally unfriendly and it is therefore desired to provide other tanning methods.

In US 3 068 190 A there is described a condensation product of formaldehyde and a sulphonated triazine derivative for non-metal tanning of pickled animal skins.

In WO 02/50313 A2 it has been proposed to add certain laccase derived enzymes to the tanning bath with synthetic or vegetable tanning agents and then to add an oxidising agent. This adds however some further step in tanning and furthermore requires a particular precise control of the enzyme activity.

In WO 94/10345 A1 there is described a metal tanning in which in a first step (a) the substrate is pre-treated with certain compounds of the there defined formula (1) containing two substituents selected from carboxy and hydroxy, and a defined reactive group, and in a second step (b) a metal tannage is applied. Pre-treatment (a) is done in order to improve tanning with metal tanning agents in subsequent step (b).

In an article by Evans et al. in J.A.L.C.A. Vol. 82 (1987) pages 88 - 95, mentioned in the introduction of WO 94/10345 A1 and which relates to a pre-treatment of certain collagen substrates with 1,2-dicarboxylic acids and subsequent aluminium tannage, there is described the treatment of lambskin with 2-N-[bis-(3',4'-dicarboxyphenyl)methyl]methylamino-4,6-dichloro-s.triazine and subsequent tannage with basified aluminium sulphate. Also in this article the pre-treatment is done in order to improve tanning with the metal tanning agent (basified aluminium sulphate) in the subsequent tanning step.

J. H. Bowes and C. W. Cater in the article "Crosslinking of Collagen" in J. Appl. Chem., 15, July, 1965, describe some crosslinking tests carried out on collagen of denatured animal tendon (kangaroo tail tendon, KTT) with various crosslinking compounds - which need not act as complete tanning materials in themselves - in particular difluorodinitrodiphenylsulphone, diisocyanates, a number of aldehydes (formaldehyde, glyoxal, malonic dialdehyde, succinic dialdehyde, glutaraldehyde, adipic dialdehyde, acrolein and dialdehyde starch), cyanuric chloride and a number of cyanuric chloride derivatives (methoxy derivative, amino derivative hydrochloride, sulphonate derivative sodium salt, 5-sulphonaphth-1-ylamino derivative sodium salt, and bis-4,4'-diaminostilbene-2,2'-disulphonic acid derivative sodium salt) - in order to assess their crosslinking potentiality, and which could be combined with conventional tanning materials for improving resistance to deterioration due to moist heat and perspiration. From their measurements, finally, they conclude that aldehydes (glutaraldehyde) appear to be most promising because of number of crosslinks and stability, whereas cyanuric chloride derivatives would be less useful as crosslinking agents in collagen.

It has now surprisingly been found that a non-metal tanning of outstanding quality can be achieved using the below defined tanning agents - in particular even as complete tanning materials - and tanning methods, which also allow to omit a pickling as conventionally carried out before chrome tanning or aldehyde tanning, unless necessary for other reasons e.g. for degreasing. The invention relates to the tanning process, the defined tanning agent compositions, the use of the tanning agents and their compositions, the tanned leather, skin or pelt and its use for further processing.

As a non-metal tanning process there is meant herein a tanning produced without using any cationic metal compounds which have tanning activity, i.e. polybasic metal compounds, mainly chromium, aluminium, iron, zirconium or titanium salts etc. As a non-metal tanning agent there is meant herein a tanning agent that does not comprise any cationic metal compounds of tanning activity, i.e. polybasic metal compounds, mainly chromium, aluminium, iron, zirconium or titanium salts, etc.

In the following, alkyl means linear or branched alkyl, if not otherwise stated.

The invention thus firstly provides a process for the production of tanned leather, skins or pelts by non-metal tanning, comprising the step of tanning a bated hide, skin or pelt with a tanning agent (A), the tanning agent (A) being at least one compound of formula (I),

R-NH-Y-SO₂-Z (I),

wherein
- R: signifies a radical of formula 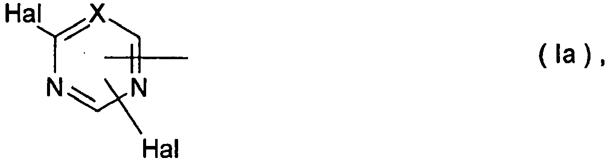 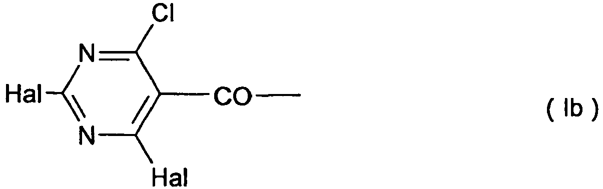 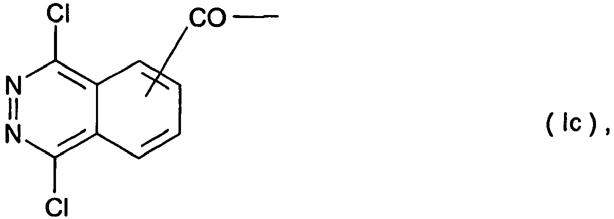 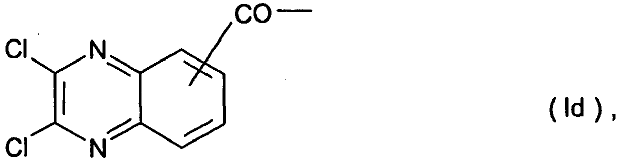 or 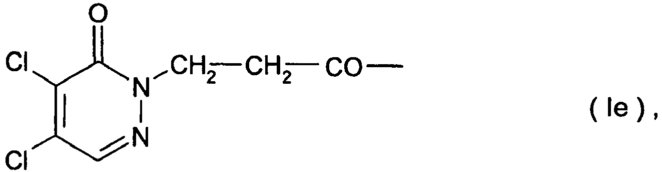
wherein
- Hal: signifies fluorine or chlorine,
- X: signifies N or CR1,
- R1: signifies hydrogen or Cl,
- Y: signifies an aliphatic, araliphatic or aromatic hydrocarbonic bridge which may optionally be interrupted by a heteroatom or heteroatomic group,
and
- Z: signifies vinyl, β-chloroethyl, β-phosphatoethyl or β-sulphatoethyl,
in a tanning bath, the tanning bath having a pH of from 6 to 10 at the beginning of tanning step.

The tanning agent (A) may consist of more than one compound of formula (I). Preferably, however, the tanning agent (A) is only one compound of formula (I).

Hal preferably signifies chlorine.

R1 preferably signifies chlorine.

X preferably signifies nitrogen or C-Cl

Y is e.g. ethylene, propylene, butylene, phenylene, mono- or dimethoxyphenylene, methyl-methoxy-phenylene, bromophenylene, sulphophenylene, naphthylene, sulphonaphthylene, propyleneaminophenylene or propyleneamidocarbonyl-phenylene, among which propylene-1,3, phenylene-1,4 and 2-sulpho-phenylene-1,4 are preferred.

Z is vinyl or a vinyl precursor which under nearly neutral to basic pH conditions yields the vinyl radical, said precursor being selected from β-chloroethyl, β-phosphatoethyl or β-sulphatoethyl, among which β-sulphatoethyl is preferred.

Any sulpho group present being expediently in the form -SO₃M, any sulphato group present being expediently in the form -OSO₃M and any phosphato group present being expediently in the form -OPO₃M₂, wherein M signifies hydrogen or an alkali metal cation or an ammonium cation, the ammonium cation being a protonated tertiary amine or a quaternary ammonium cation,

Where M is an alkali metal cation or a ammonium cation, then it may be any alkali metal cation or an ammonium cation as conventionally employed for salt formation in anionic compounds.

Preferably, the alkali metal cation is selected from the group consisting of lithium, sodium and potassium, more preferably the alkali metal cation is sodium. Preferably, the ammonium cation is a cation of formula (Ib), 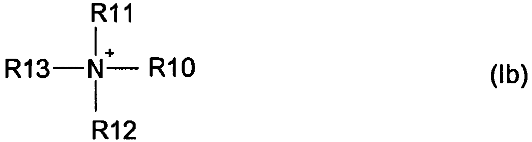 wherein
- R10, R11, R12 and R13: are same or different and independently from each other selected from the group consisting of H, C₁₋₄-alkyl, C₂₋₃-hydroxyalkyl and benzyl, with the proviso, that only one of the four substituents R10, R11, R12 and R13 may be H.
Preferably,
- R10: is H or C₁₋₄-alkyl, and
- R11, R12 and R13: are same or different and independently from each other selected from the group consisting of C₁₋₄-alkyl, C₂₋₃-hydroxyalkyl; or
- R10, R11, R12 and R13: are same or different and independently from each other are C₁₋₄-alkyl; or
- R10, R11 and R12: are same or different and independently from each other are C₁₋₄-alkyl or C₂₋₃-hydroxyalkyl, and
- R13: is benzyl.

More preferably, the ammonium cation is selected from the group consisting of monohydrogen-tri(C₂₋₃-alkanol)-ammonium, tetra (C₁₋₄-alkyl)-ammonium, tri(C₁₋₄-alkyl)-mono(C₂₋₃-alkanol)-ammonium, di(C₂₋₃-alkanol)-di(C₁₋₄-alkyl)-ammonium, mono(C₁₋₄-alkyl)-tri(C₂₋₃-alkanol)-ammonium, monobenzyl-tri(C₁₋₄-alkyl)-ammonium and monobenzyl-tri(C₂₋₃-alkanol)-ammonium.

Especially, the ammonium cation is a quaternary ammonium cation.

More especially preferably, M is an alkali metal cation, even more especially preferably M is sodium.

Among the radicals of formulae (Ia) to (Ie) those of formula (Ia) are preferred.

Preferably, compound of formula (I) is a compound of formula (I-I), 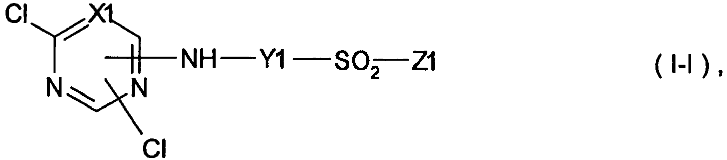 wherein
- X1: is nitrogen, CH or CCI,
- Y1: is C₂₋₄-alkylene, phenylene or sulphophenylene,
and
- Z1: is vinyl or β-sulphatoethyl,
more preferably a compound of formula (I-II). 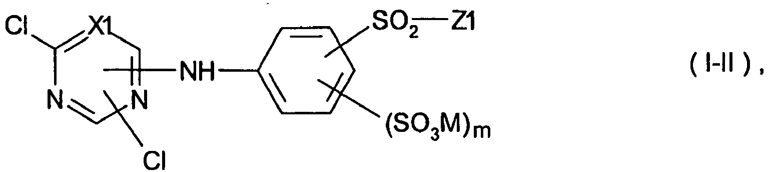 wherein m is 0 or 1.

The compounds of formula (I) are known or may be produced according to known methods, preferably by reaction of a compound of formula (II),

R-Hal (II)

in which Hal preferably is chlorine,
with a compound of formula (III),

H₂N-Y-SO₂-Z (III),

with R, Y and Z having the definition as given above, also with all their preferred embodiments.

The compounds of formulae (II) and (III) are known.

The reaction of the compounds of formula (II) with the compounds of formula (III) is a reaction which splits off an acid H-Hal.

The reaction of the compound of formula (II) with the compound of formula (III) may be carried out in an aqueous, aqueous/organic or organic medium. Where R is a radical of formula (Ia) the medium is preferably aqueous. Where R is a radical of any of the formulae (Ib) to (Ie) the reaction medium is preferably organic.

Where R is a radical of formula (Ia), preferably, an aqueous solution or dispersion of compound of formula (III) is mixed with the compound of formula (II). The compound of formula (II) is preferably in form of a dry compound, an organic solution or dispersion or an aqueous dispersion. Preferably, an aqueous solution or dispersion of compound of formula (III) is added to an aqueous dispersion of compound of formula (II). In another preferred embodiment, the dry compound of formula (II) is stirred into a preferably aqueous solution or dispersion of compound of formula (III).

Where R is a radical of any of the formulae (Ib) to (Ie) an aqueous solution or dispersion of compound of formula (III) is mixed with the compound of formula (II). The compound of formula (II) is preferably in form of a dry compound or an organic solution or dispersion. Preferably, an aqueous solution or dispersion of compound of formula (III) is added to an organic solution of compound of formula (II).

Suitable organic media include e.g. acetone, methylethylketone, dimethylsulphoxide, tetrahydrofuran, xylene and toluene.

Preferably, 1.00 ± 0.05 mol of compound of formula (II) per mol of compound of formula (III) is used.

The concentration of compound of formula (II) is e.g. of from 2 to 70 % by weight, preferably 5 to 50 % by weight, the % by weight being based on the total weight of the reaction mixture comprising compound of formula (II), compound of formula (III) and the aqueous, aqueous/organic or organic medium.

When the compound of formula (II) is dissolved in an organic medium, its concentration is preferably high, in particular close to saturation, in order to reduce to a minimum the amount of solvent to be evaporated upon completion of the reaction.

Dispersion of compound of formula (II) or of compound of formula (III), preferably for dispersion in water, may be brought about by plain stirring or also by the use of a suitable surfactant (B) acting as a dispersing agent.

For dispersing a compound of formula (II) or a compound of formula (III), wherein a sulphonic acid group, a sulphato group or a phosphato group of a compound of formula (III) is in protonated form, preferably for dispersing in water, surfactant (B) may be employed in a suitable efficient concentration, e.g. in a weight ratio of surfactant (B) to the compound of formula (II) or to the compound of formula (III) preferably of from 0.002 to 2, more preferably of from 0.004 and 1, even more preferably of from 0.005 and 0.5.

Compounds of formula (III) containing the above mentioned anionic groups may be used in salt form, the salt form being preferably an alkali metal salt of the sulphonic acid, sulphato- or phosphato group, more preferably a sodium salt. The compounds of formula (III) in salt form are in general soluble in water and they are suitably employed in the form of an aqueous solution or dispersion (at concentrations higher than the one corresponding to the saturated solution), preferably in an amount of from 2 to 70 % by weight, more preferably 10 to 50 % by weight, the % weight being based on the total weight of the solution or dispersion of compound of formula (III). Preferably this solution or dispersion contains also a dispersing agent (B) as mentioned above, in a concentration as mentioned above suitable for dispersing compound of formula (II) when the latter is added as a dry product and is stirred into the solution.

According to a preferred embodiment, a compound of formula (II) or an organic solution of a compound of formula (II) is stirred into an aqueous or organic solution of a compound of formula (III) containing a surfactant (B).

The surfactant (B) is preferably selected from the group consisting of
(B1) non-ionic surfactant,
(B2) anionic surfactant,
(B3) cationic surfactant,
(B4) amphoteric surfactant and
mixtures thereof,
with the proviso that the surfactant (B) does not have a substituent capable of reacting with the compound of formula (II) under the chosen reaction conditions, in particular does not have a primary or secondary amino group. Preferable mixtures are mixtures of (B2) with (B1) and/or (B4), of (B3) with (B1) and/or (B4) or of (B1) with (B4).

Preferably, the non-ionic surfactant (B1) is selected from the group consisting of oligo- or polyglycolethers of aliphatic alcohols, oligo- or polyglycolesters of aliphatic carboxylic acids, oxyalkylated fatty acid amides and oxyalkylated partial esters of glycerol or sorbitol with fatty acids.

Preferably, the oxyalkylation of the fatty acid amides and the oxyalkylation of the partial esters of glycerol or sorbitol with fatty acids leads to oligo- or polyglycolether chains.

Preferably any oligo- or polyglycolether chain contains 2 to 60, more preferably 2 to 24 oxyalkylene units which are oxyethylene and optionally oxypropylene units, and preferably at least 40 mol-%, more preferably at least 50 mol-% being oxyethylene units and preferably, the non-ionic surfactant (B1) contains at least two oxyethylene units.

Preferably, the lipophilic aliphatic radical in the aliphatic alcohol, aliphatic carboxylic acids, fatty acid amides and fatty acids contain 8 to 24 carbon atoms. The aliphatic radical may be saturated or unsaturated (preferably it contains only one double bond) and may be linear or branched, the branched preferably saturated.

As examples of aliphatic alcohols there may be mentioned lauryl, cetyl, myristyl, stearyl or oleyl alcohol, and C₉₋₁₅-oxoalcohols.

As examples of aliphatic carboxylic acids and of fatty acids amides there may be mentioned lauric, palmitic, myristic, stearic, behenic, arachic or oleic acid or amide.

The oligo- or polyglycolethers of aliphatic alcohols may be produced by oxyethylation and, if oxypropylene units are also to be present, oxypropylation of the corresponding aliphatic alcohols.

The oxyalkylated fatty acid amides may be produced e.g. by oxyethylation, and if oxypropylene units are also to be present, by oxypropylation of the corresponding fatty acid amides, e.g. of aliphatic acid diethanolamide or diisopropanolamide.

Oligo- or polyglycolesters and sorbitol monoesters may e.g. be produced by esterification of a corresponding oligo- or poly-ethylene- and optionally -propylene-glycolether or sorbitol. Monoglycerides may be partial saponification products of corresponding naturally occurring triglycerides.

Preferably, the anionic surfactant (B2) is selected from the group consisting of anionic polycarboxylates, aliphatic fatty acids in salt form (soaps), methyltaurides of aliphatic fatty acids and anionic derivatives of non-ionic surfactants, preferably of non-ionic surfactants (B1), in particular carboxymethylation products or carboxyethylation products of non-ionic surfactants (B1) or sulphuric acid monoesters or phosphoric acids monoesters of non-ionic surfactants (B1), in particular in alkali metal salt form.
Preferred anionic polycarboxylates are polyacrylates and polymethacrylates.

Preferably, the cationic surfactant (B3) is selected from the group consisting of tertiary or preferably quaternary derivatives of fatty amines, e.g. with 8 to 24 carbon atoms in the fatty amine chain, and in which the substituents of the tertiary amino group or quaternary ammonium group are C₁₋₄ alkyl (preferably methyl or ethyl) or hydroxyl-C₁₋₄-alkyl (preferably ethanol or isopropanol) and optionally benzyl, and where, if desired, the tertiary amino group or quaternary ammonium group may also contain an oligo- or polyglycolether chain analogously as mentioned above in the non-ionic surfactants (B1). As examples of fatty amines there may be mentioned lauryl, cetyl, myristyl, stearyl or oleyl amine and the amino group may be substituted with two methyl or ethyl groups and optionally a methyl or benzyl group, or with three methyl or ethyl groups or with two ethanol groups. If the tertiary amino group or quaternary ammonium group is oxyalkylated it may preferably contain a total of 2 to 40, more preferably 2 to 24 alkylenoxy groups, preferably at least 40 mol-% of which are ethyleneoxy and the remaining are propylenoxy. Tertiary amino groups are preferably protonated e.g. with hydrochloric, phosphoric or C₂₋₂₀-, preferably C₂₋₅-alkanoic acid.

Preferably, the amphoteric surfactants (B4) are anionic derivatives of (B3), e.g. carboxymethylation products of (B3), carboxyethylation products of (B3), sulphuric or sulphamic acid monoesters of (B3), or phosphoric acid mono- or diesters of (B3) of those cationic surfactants (B3) containing a hydroxy group, betaines and sulphobetaines.

Preferably, the surfactant (B) is a non-ionic surfactant (B1).

Preferably non-ionic surfactants (B1) are used, more preferably the non-ionic surfactants (B1) are compounds of formula (IV) (polyglycolethers),

R2-O-(C₂₋₃-alkylene-O)ᵣ-H (IV)

wherein
- R2: signifies C₈₋₂₄-alkyl or C₈₋₂₄-alkenyl,
- C₂₋₃-alkylene: is selected from the group consisting of -CH₂-CH₂-, -CH₂-CH(CH₃)-, -CH(CH₃)-CH₂- and combinations thereof, preferably of -CH₂-CH₂-, -CH₂-CH(CH₃)- and combinations thereof,
- r: signifies 2 to 60,

in case that the non-ionic surfactant (B1) of formula (IV) consists of more than one compound of formula (IV), r may also be calculated and expressed as the average number of radicals of the formula (IV) in the mixture,
with the proviso that the compound of formula (IV) contains at least two ethyleneoxy units, and with the proviso that at least 40 % of the total number of alkyleneoxy units in the compound of formula (IV) are ethyleneoxy.

The reaction of the compound of formula (II) with the compound of formula (III) is preferably done at a temperature below 40 °C, more preferably of from -10 to 40 °C, even more preferably of from -10 to +25 °C, especially preferably of from 0 to 15 °C.

Preferably, the reaction of the compound of formula (II) with the compound of formula (III) is carried out under acidic to weakly basic pH conditions, more preferably at a pH of from 2 to 9, even more preferably under acidic to nearly neutral conditions, especially preferably at a pH of from 2.5 to 7, as suitable for reacting the specified halogen of the compound of formula (II).

The reaction is preferably carried out in the presence of a base or other reactant suitable for binding the acid H-Hal.

The base or other reactant which can be used for binding the acid H-Hal and which can be used for pH adjustment during or/and after the reaction of the compound of formula (II) with the compound of formula (III), is preferably selected from the group consisting of alkali metal base, quaternary ammonium hydroxide and carbonate.

The alkali metal base is preferably selected from the group consisting of hydroxide, carbonate and bicarbonate of lithium, sodium and potassium and mixtures thereof.

The quaternary ammonium hydroxide and carbonate is preferably selected from the group consisting of hydroxide and carbonate of tetramethyl-ammonium, tetraethyl-ammonium and benzyl-trimethyl-ammonium.

If an organic solvent has been used, this may be eliminated by evaporation and, if desired, the resulting product may be dissolved or dispersed in water. If a dry product is desired, this may be obtained from an aqueous solution or dispersion of the reaction product in a manner conventional per se, e.g. by precipitation (e.g. by salting out) and filtration, or by evaporation under controlled conditions.

A tanning agent (A) comprising more the one compound of formula (I) can be produced by using more than one compound of formula (III) in the reaction of compound of formula (II) with compound of formula (III), or by physical mixing the compounds of formula (I). The physical mixing can be done in dry form and/or in dissolved or dispersed form, preferably in dissolved or dispersed form in water.

The tanning agent (A) may be used as such, as produced, preferably in dry form or more preferably in the form of an aqueous solution or dispersion.

According to a particular aspect of the invention, the tanning agent (A) is in the form of an aqueous composition (T) which is free of any metal-based compounds which have tanning activity, aqueous composition (T) comprising the tanning agent (A) and water, and preferably comprises in addition the surfactant (B).

Therefore, another subject of the invention is a tanning process as described above, also with all its preferred embodiments, wherein the tanning agent (A) is employed in the form of an aqueous composition (T) which is free of metal compounds of tanning activity.

More preferably, the composition (T) comprises of from 2 to 70 % by weight, especially 10 to 50 % by weight, the % by weight being based on the total weight of the composition (T), of tanning agent (A).

Where surfactant (B) is present in the composition (T), the weight ratio of surfactant (B) to tanning agent (A) in the composition (T) is preferably of from 0.001 to 1, more preferably of from 0.002 to 0.4, even more preferably of from 0.005 to 0.1.

Preferably, in the composition (T), the surfactant (B) is the non-ionic surfactant (B1). If an anionic, cationic or amphoteric surfactant (B2), (B3) or (B4) is used, its amount is preferably of from 0.001 to 10 mol-%, the mol-% with respect to the total molar amount of tanning agent (A). More preferably the surfactant (B) is only the non-ionic surfactant (B1). If surfactant (B2) is a polycarboxylate, its amount is preferably < 5 % by weight, more preferably of from 0.01 to 4 % by weight, even more preferably from 0.05 to 2 % by weight, the % by weight being always based on the total weight of tanning agent (A).

The composition (T) has preferably an acidic to neutral pH, more preferably it has an acidic pH. For pH adjustment, a suitable buffer may be employed and composition (T) thus preferably further comprises a buffer (C1) to keep an acidic to neutral pH, preferably to keep an acidic pH, more preferably to keep a pH of from 1 to 7, even more preferably to keep a pH of from 1 to 5.

The buffer (C1) is preferably selected from the group consisting of phthalate, oxalate and citrate of sodium and/or of potassium, mono- and di-hydrogenphosphate of sodium and/or of potassium, mixture of phosphoric acid and di-hydrogenphosphate of sodium and/or potassium and mixtures thereof, preferably a combination of KH₂PO₄ or NaH₂PO₄ and Na₂HPO₄ or K₂HPO₄.

The amount of buffer (C1) in the composition (T) is preferably chosen so as to achieve the desired pH mentioned above. The amount of buffer (C1) is preferably of from 0.1 to 5 % by weight, the % by weight being based on the total weight of the composition (T).

Compositions (T) advantageously may further comprise
an agent (D) to protect against the damaging action of microorganisms, preferably, agent (D) is a bacteriostatic additive and/or a microbicide, e.g. a fungicide.

As agent (D) there may be employed commercially available products, which may be employed in small concentrations, in particular according to the commercially recommended ones. The amount of of agent (D) in the composition (T) is preferably of from 0 to 10 % by weight, more preferably of from 0.01 to 10 % by weight, even more preferably of from 0.02 to 1 % by weight, the % by weight being based on the total weight of the composition (T).

According to a particular further feature of the invention, aqueous compositions (T) may further comprise
a polysaccharide- based thickener (E).

As thickener (E) there may be employed products known per se, in particular gums, carbohydrates, carbohydrate derivatives, e.g. pectins and hydrophilic cellulose derivatives, which with water form viscous solutions (colloidal or true solutions). There may be mentioned gums as obtainable by fermentation and optionally chemical modification of natural plant-exudates, e.g. xanthan gum, tragacanth gum, guar gum, carrageenan gum, alginate gum, agar gum, gum ghatti, and water soluble carbohydrate derivatives in particular pectins, e.g. pectins from fruits (e.g. citric fruits or apples) and amylopectins (e.g. from corn starch or potato starch), and hydroxyethylcellulose. The gums, carbohydrates and carbohydrate derivatives may also be chemically modified, provided that they do not contain any substituents capable of reacting with tanning agent (A) under storage or application conditions, in particular they do not contain any basic amino groups, especially any primary or secondary amino groups.

Thickener (E) may be employed in a minor proportion in the composition (T), in particular as suffices for adjusting the viscosity of (T) so that it is still flowable. Where thickener (E) is employed in the composition (T), it is added preferably in such a concentration that the viscosity of the composition (T) at 20 °C is preferably ≤ 50,000 mPa·s, more preferably of from 60 to 15,000 mPa·s, even more preferably of from 70 to 10,000 mPa·s. The viscosity is the Brookfield rotational viscosity, determined according to standard method ASTM D 2669.

Preferably the amount of thickener (E) in the composition (T) is of from 0 to 5 % by weight, more preferably of from 0.1 to 5 % by weight, the % by weight being based on the total weight of the composition (T).

Preferred compositions (T) are compositions (T1) comprising, in addition to the tanning agent (A), surfactant (B) and/or buffer (C1), more preferably surfactant (B) or surfactant (B) and buffer (C1); preferably compositions (T1) further comprise an agent (D) and/or a thickener (E).

Therefore, another preferred embodiment of the invention is a tanning process as described above, also with all its preferred embodiments, wherein the composition (T) is an aqueous composition (T1) comprising the tanning agent (A) and further comprising a surfactant (B) and/or a buffer (C₁) to keep an acidic to neutral pH.

Therefore, another preferred embodiment of the invention is a tanning process as described above, also with all its preferred embodiments, wherein the composition (T) is an aqueous composition (T1) of a tanning agent (A) comprising a surfactant (B) and/or a buffer (C₁) and further comprising an agent (D) to protect against the damaging action of microorganisms and/or a polysaccharide-based thickener (E).

Preferably, the composition (T) is a composition (T1) comprising
of from 2 to 70 % by weight, preferably 10 - 50 % by weight, of tanning agent (A), the % by weight being based on the total weight of the composition (T1); surfactant (B) in a weight ratio of surfactant (B) to tanning agent (A) of from 0.001 to 1, more preferably of from 0.002 to 0.4, even more preferably of from 0.005 to 0.1;
buffer (C1) in such an amount as to achieve a pH in the composition (T1) of from 1 to 7, more preferably pH 1 to 5, preferably the amount of buffer (C1) is of from 0.1 to 5 % by weight, the % by weight being based on the total weight of the composition (T1);
of from 0 to 10 % by weight, preferably of from 0.01 to 10 % by weight, more preferably of from 0.02 to 1 % by weight, of agent (D), the % by weight being based on the total weight of the composition (T1),
thickener (E) in such an amount that the viscosity of the composition (T1) at 20 °C (Brookfield rotational viscosity measured according to standard method ASTM D 2669) is ≤ 50,000 mPa·s, preferably of from 60 to 15,000 mPa·s, more preferably of from 70 to 10,000 mPa·s, especially thickener (E) is used in an amount of from 0 to 5 %, more preferably of from 0.1 to 5 % by weight, the % by weight being based on the total weight of the composition (T1);
and the dry substance content of the composition (T1) is preferably of from 4 to 75 % by weight, more preferably in the range of 10 to 60 % by weight, the % by weight being based on the total weight of the composition (T1).
The dry substance content of (T) or (T1) may be assessed in a manner conventional per se, e.g. by calculation based on the employed reactants and components - mostly by simple addition of the amounts of the substances (expressed in dry form) added for the production of (T) or (T1) and substracting any water formed during the reaction -, or, which is the preferable way, by substracting the water content determined in a conventional way, e.g. by titration,e.g. Karl Fischer titration, from the total weight of (T) or (T1).

Particularly preferably, composition (T) or (T1) is a composition (T2) which contains the thickener (E), preferably in an amount of ≥ 0.1 % by weight, more preferably in the range of from 0.1 to 5 % by weight of (E), the % by weight being based on the total weight of the composition (T2). The viscosity of the composition (T2) at 20 °C (Brookfield rotational viscosity measured according to standard method ASTM D 2669) is preferably in the range of 60 to 15,000 mPa·s, more preferably 70 to 10,000 mPa·s.

This composition (T2) is of satisfactory stability and is suitable for storage and shipment and is directly usable. It is readily dilutable with water and may be directly metered into the tanning drum, if desired.

As a substrate for the treatment with tanning agent (A), in particular for tanning, there may be used any conventional animal hides, skins and pelts as are in general employed for tanning, e.g. hides from cow, calf or buffalo (e.g. also as split hides), skins from goat, sheep or pig, buckskins and pelts; but also other hides and skins e.g. from other mammals (foal, camel, lama, kangaroo, wallaroo, wallaby), reptiles (snakes, lizards), fishes (shark) or birds (ostrich), woolled skins and furskins, may be used in the process of the invention.

The bated substrates (animal hides, skins or pelts) may have been processed in the beamhouse before tanning, i.e. trimmed, soaked, limed, delimed and bated in conventional way. Before deliming the limed hides, skins or pelts are usually fleshed and, if required, split and optionally scudded, shaved etc. and, if required, defatted and/or dehaired.

Bated hides, skins and pelts to be used as substrates in the process of the invention may have been produced in conventional way, in the beamhouse, in particular by deliming the limed substrates and bating, using known agents for each of the mentioned processing steps.

Deliming may have been carried out in conventional way with known compounds such as acids, ammonium salts of low molecular aliphatic carboxylic acids, ammonium sulphate or sodium phosphate. Optionally the deliming composition may contain an enzyme e.g. as mentioned below, so that, if desired, bating and deliming may at least in part be combined.

For bating there may be employed known proteolytic bates, in particular in the form of bating compositions based on conventional proteolytic enzymes, mainly bacterial proteases, fungal proteases, and pancreas enzyme. Occasionally also other enzymes may be employed, such as lipases, amylases and also other hydrolases. Pancreas enzyme alone or in admixture with other enzymes (e.g. lipases, amylases and also other hydrolases) is preferred. Commercial forms of such enzymes may be formulated together with other components, especially with some mineral carriers, saccharides or polysaccharides and/or a hydrotrope. For the purpose of the invention substrates conventionally bated with bating compositions based on pancreas enzyme are well suitable.

The above bating compositions are in particular of an optimum activity in the weakly basic pH range, more particularly at a basic pH ≤ 11, and consequently the pH of the bated substrate is preferably in the weakly basic range, in particular a pH in the range of 7.5 to 11, more preferably 7.5 to 10.

Where the substrate has been delimed with acids, also acidic bates may be used, e.g. pepsins e.g. in the form of a solution of 2 % pepsin in water and at a pH in the range of 3 - 4.

The tanning process of the invention is based on a true tanning with the tanning agent (A) leading to leathers, skins and pelts with characteristic true tannage properties, such as a reduction or elimination of swellability, reduction of deformability and augmentation of firmness, diminution of shrinkage in volume, surface and thickness by drying, and increment of the porosity of the fibre texture, and further rising of shrinkage temperature and fastness of the collagen fibre to hot water, and of being non-rotting.

As a "step" in the tanning process according to the invention there is meant any tanning step in a tanning process in which the tanning agent (A) acts on the non-tanned or not fully tanned substrate, i.e. pre-tanning, main tanning, or full or complete tanning (including also a combined tanning). The tanning agent (A) can thus be employed for pre-tanning, for main tanning, or for full (i.e. complete) tanning or for both pre-tanning and main tanning, and for combined tannings. The use of the tanning agent (A) as a full tanning agent or as both a pre-tanning agent and a main tanning agent is however the most relevant aspect of the invention.

The tanning process of the invention - which may be a one stage tanning, i.e. a full tanning, or a two stage tanning, i.e. a pre-tanning followed by a main tanning, or a combined tanning - can be carried out directly after bating.

The tanning process with the tanning agent (A) of the invention may be carried out in an aqueous, aqueous/organic or organic medium; suitable organic media include e.g. ethanol, isopropanol, acetone, methylethylketone, dimethylsulphoxide, chloroform, chlorobenzene and toluene. Preferably it is carried out in an aqueous bath, e.g. at a bath length of 30 to 400 % by weight of water, preferably 40 to 200 %, most preferably 40 to 100 %, the % by weight based on the weight of the fleshed or (if the hide has been split) the split substrate, and at temperatures preferably of from 10 to 50 °C, more preferably of from 10 to 40 °C, even more preferably of from 15 to 40 °C. Preferably tanning is begun at a temperature of from 10 to 35 °C, more preferably of from 15 to 30 °C, and at the end the temperature is allowed to rise preferably by 5 to 20 degrees, more preferably by 8 to 15 degrees, to an end temperature of from 20 to 40 °C, preferably of from 25 to 40 °C.

For the tanning process of the invention the tanning agent (A) is added in the tanning bath in an efficient concentration, preferably of from 0.5 to 20 % by weight, more preferably of from 1 to 10 % by weight, the % by weight being based on the weight of the fleshed or split substrate. The tanning agent (A) may be added in dry form or preferably in the form of an aqueous composition, preferably as mentioned above as a composition (T).

With particular preference a surfactant, in particular as mentioned above a surfactant (B), preferably a non-ionic surfactant (B1), and/or a buffer (C2) for nearly neutral to basic pH values, in particular pH ≥ 6, may be added in the tanning bath, in a weight ratio as suitable in order to achieve the desired pH at the beginning of the tanning step.

Therefore, another preferred aspect of the invention is a tanning process as described above, also with all its preferred embodiments, wherein the tanning bath comprises a buffer (C2) to achieve a nearly neutral to basic pH at the beginning of the tanning step.

As buffers (C2) there may be employed known buffers, preferably selected from the group consisting of sodium and/or of potassium bicarbonate, sodium and/or of potassium carbonate, sodium and/or of potassium hydrogen phosphate, sodium borate and trishydroxymethylaminomethane. Preferably buffer (C2) is a combination of KH₂PO₄ or NaH₂PO₄ and K₂HPO₄ or Na₂HPO₄. For the tanning process of the invention it is of particular advantage to employ compositions (T) as described above, which preferably already contain a surfactant (B) and optionally also agent (D) and/or thickener (E). The buffer (C2) may be added directly into the tanning bath. Preferably the buffer (C2) is added in a two-stage tanning before the main tanning step in order to set the pH of the main tanning bath. Compositions (T), in particular compositions (T1), are readily efficient for tanning. The composition may contain some salt as resulting as a by-product from the synthesis of the compound of formula (I) from the reaction of compound of formula (II) with compound of formula (III).

The tanning process of the invention is started at a pH of from 6 to 10, preferably of from 6 to 9, more preferably of from 6.5 to 8.5, in particular as suitable for inducing the reaction of tanning agent (A) with the substrate.

During the tanning, the pH gradually decreases spontaneously by a few pH units, in particular by from 1 to 4 pH units, to a pH in the nearly neutral to weakly acidic pH range, in particular to a pH of from 7 to 3.5, preferably of from 6.5 to 3.5. The process may thus be carried out under self-regulating pH conditions. If desired, however, the tanning reaction may be influenced by the addition of a minor proportion of acid (e.g. a mineral acid, e.g. sulphuric or phosphoric acid, or a low molecular carboxylic acid e.g. with 1 to 4, preferably 1 or 2, carbon atoms, e.g. formic or acetic acid), or by the addition of a minor proportion of base (alkali), e.g. in order to accelerate or decelerate the reaction and/or to shift the pH slightly towards more neutral values.

According to a preferred feature, where Z contains a predecessor of vinyl, i.e. a sulphatoethyl, phosphatoethyl or chloroethyl group, at the end of the tanning step the pH is increased to alkaline values, e.g. to pH 8-10, by addition of a base, preferably sodium carbonate, in order to favour completion of the reaction of the sulphato- or phosphato group or of the the chlorine, e.g. during 1 - 5 hours preferably 1.5 to 3 hours, and then the pH is increased to acidic values e.g. pH 2-5, preferably 3.5 to 4.5, for further completion of the tanning, e.g. during 6 - 12 hours.

In the process of the invention pickling is in principle not necessary and may mostly be omitted. Therefore, subject of the invention is also a tanning process as described above, also in all its preferred embodiments, wherein the bated hide or skin or pelt is subjected to tanning with a tanning agent (A) without previous pickling.

If desired, however, e.g. in order to obtain a certain consistency of the substrate or if the substrate is to be defatted under acidic conditions before tanning, a pickling may be carried out. The pickled substrate may be defatted in conventional way. Sometimes commercially available hides have already been pickled. If the substrate has been pickled, it is suitably depickled before tanning in order to achieve the desired pH in the range of 6 to 10. Therefore, another subject of the invention is a tanning process as described above, also with all its preferred embodiments, wherein a bated and pickled hide, skin or pelt is depickled to a pH in the range of 6 to 10 before tanning with a tanning agent (A).

A depickling may be carried out as suitable to achieve pH values in the range of 6 to 10, preferably 6 to 9, by employing a corresponding quantity of base for depickling. Depickling may be carried out in conventional manner using known compounds, e.g. sodium and/or potassium bicarbonate or/and formate, and under conditions conventional per se, e.g. at bath lengths in the range of 50 to 400 % by weight of water, the % by weight being based on the weight of the fleshed or (if the hide has been split) the split substrate, at temperatures in the range of 10 to 30 °C and rotation in the drum for 60 minutes to 6 hours. If desired, e.g. to ensure depickling also in the interior of the substrate, the substrate may be kept in the depickling bath e.g. by dwelling overnight, optionally with intermittent drum rotation, e.g. 5 to 15 minutes every hour.

If the tanning process is started at nearly neutral conditions, in particular at a pH in the range of 6 to 7.5, - especially when using pickled and depickled substrates-the pH may initially also be kept in this range or increased, to give a pH in the range of 6 to 9, by addition of a base.

If - as preferred - no pickling and depickling is carried out, pretanning or full tanning may be carried out directly on the bated substrate. The pH at the beginning of the tanning process is preferably nearly neutral to basic, in particular in the range of 6.5 to 10, preferably 6.8 to 9, and during the treatment gradually decreases spontaneously by a few pH units, in particular by 1 to 4 pH units, to a nearly neutral to weakly acidic pH range, in particular 7 to 4, preferably 6.5 to 4.5. The temperature is preferably in the range of 10 to 40 °C, more preferably 15 to 35 °C. More particularly tanning is preferably begun at 10 to 30 °C more preferably 15 to 25 °C, and at the end the temperature is allowed to rise by 5 to 20 degrees, preferably by 8 to 15 degrees, to 20 to 40 °C, preferably 25 to 40 °C.

The tanning process of the invention is very simple and may be carried out in a relatively short time, in particular within about 5 to 24 hours, e.g. 6 to 12 hours.

After tanning the exhausted tanning bath may be drained off and the tanned leather, skin or pelt may be rinsed or washed e.g. one to three times with water (e.g. 100 to 600 % by weight of water, preferably 250 to 400 % by weight of water, the % by weight being based on the weight of the fleshed or (if the hide has been split) the split substrate), to which if desired some conventional surfactants may be added, in order to favour slippage of the grain. If desired also a biocide e.g. as mentioned above for (D), may be added during the process, e.g. into the last washing bath, as a preserving agent for the resulting tanned hide or skin or pelt.

If desired a further non-mineral tanning agent (F), which is different from (A), of anionic or/and ethylenically unsaturated character or/and containing groups of basic character may be applied before, after or together with tanning agent (A) in pre-tanning, in main tanning or in full tanning, preferably for pre-tanning before a main tanning with (A), or in combination with (A) in a main or full tanning, or/and preferably for a complementary tanning after a main or full tanning with (A), or even for retanning.
(F) preferably is selected from the group consisting of
   (F1) a vegetable tanning agent,
   (F2) a syntan,
   (F3) a synthetic, semisynthetic or natural resin or polymer,
   (F3) a synthetic, semisynthetic or natural resin or polymer,
   (F4) a tanning natural oil or modified oil,
   (F5) a compound of formula (V) 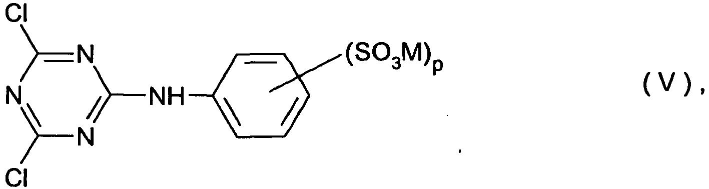 wherein p is 1 or 2
      and M is as defined above,
   (F6) a tanning oxazolidine, in particular of formula (VI) or (VII) 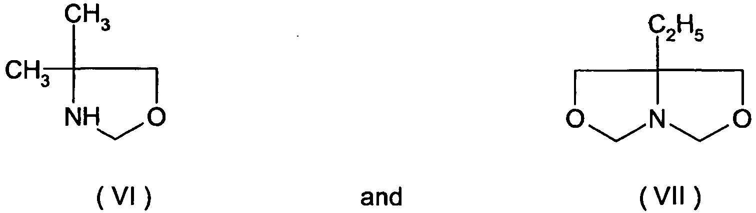 and
      mixtures of two or more thereof.
      mixtures thereof.

As vegetable tanning agents (F1) there may be employed known vegetable tanning agents, in particular pyrogallol- or pyrocatechin-based tannins, e.g. valonea, mimosa, teri, tara, oak, pinewood, sumach, quebracho and chestnut.

As syntans (F2) there may be employed known synthetic tanning agents, in particular syntans derived from sulphonated phenols and/or naphthols, and/or sulphones or polymers of sulphones and/or sulphonated phenols and/or sulphonated naphthols with formaldehyde or acetaldehyde and optionally urea, among which sulphone-based products are preferred.

As synthetic or semisynthetic or natural resins or polymers (F3) there may be employed e.g. known polyacrylates, polymethacrylates, copolymers of maleic anhydride and styrene, condensation products of formaldehyde with melamine or dicyandiamide, lignins and natural flours. Among the synthetic or semisynthetic or natural resins or polymers (F3), those of anionic character (e.g. polyacrylates, polymethacrylates, lignin sulphonates and copolymers of maleic anhydride and styrene) and which are free of basic amino groups are designated herein as (F3-I).

As natural or modified oils (F4) there may be employed known natural triglycerides, e.g. rape seed oil, fish oils or their oxidised derivatives, sulphated, sulphonated or oxy-sulphited fish oils, or their oxidised derivatives, or surrogates thereof.

As compounds (F5) there may be employed compounds in which p is 1 or 2, or mixtures compounds in which p is 1 and p is 2.

Tanning with (A) may be carried out as a full tanning, or as a pre-tanning before a non-metal main tanning, which may be carried out with a vegetable tanning agent or with a synthetic tanning agent other than (A) - e.g. as mentioned above as (F) - or also with a tanning agent (A) according to the invention, or as a main tanning after a non-metal or even non-mineral pre-tanning (which may be vegetable or synthetic) e.g. carried out with (F) mentioned above. Where the tanning with (A) of the invention is carried out as a main tanning subsequently to a vegetable pretanning or to a synthetic pre-tanning with syntans, the pH may if required be adjusted to the desired value between 6 and 10, e.g. by addition of an alkali metal carbonate, bicarbonate or formate for the tanning method of the invention.

According to a particular feature of the invention, the tanning agent (A) may be used in combination with another non-mineral tanning agent (F), preferably (F-I), (F-I) being selected from the group consisting of (F1), (F2) and (F3-I), e.g. in a weight ratio of (A) to (F-I) being of from 0.05 to 20, more particularly from 2 to 10. The concentration of the combined tanning agents may be as desired for achieving a defined tanning, e.g. of from of 0.5 to 20 % by weight, preferably 1 to 10 % by weight, the % by weight based on the weight of the fleshed substrate.

According to a further particularly preferred feature of the invention, the substrates are first tanned in one or two stages with (A) and then are subjected to a complementary tanning with a non-mineral tanning agent (F), which preferably is (F-II), (F-II) being selected from the group consisting of (F1), (F2), (F3) and (F5). As a complementary tanning there is intended here an additional tanning step carried out after main or full tanning with (A), and which substantially does not modify the characteristic kind of properties of the leather, skin or pelt tanned with (A), but may improve some of the typical tannage properties. Typically it is carried out with a smaller amount of the complementary tanning agent (F) compared with the amount of the employed main or full tanning agent (A), preferably 5 to 80 % by weight, preferably 10 to 60 % by weight of (F), the % by weight based on the weight of the employed amount of (A). This complementary tanning may advantageously be carried out sequentially to the tanning with (A) under temperature conditions as mentioned above, e.g. 10 to 40 °C, at bath lengths preferably as used for tanning with (A), e.g. in the range of 40 to 200 % by weight of water, the % by weight being based on the weight of the fleshed or (if the hide has been split) the split substrate, and under pH conditions as resulting from the tannage with (A), preferably after rinsing with water, usually this pH may range in the scope of 4 to 7.

Complementary tanning with (F), preferably with (F-II), may be carried out in the tannery directly after tanning, or even after having rinsed, dried and optionally mechanically treated the tanned leather, skin or pelt.

At the end of the tanning process, after draining off of the exhausted tanning or complementary tanning bath, the tanned leather, skin or pelt may - if desired - be treated with one or more conventional additives e.g. one or more surfactants, preferably as mentioned above as (B), mainly (B1) or (B3), for protecting the grain from damaging friction, or/and with a preserving agent, preferably as mentioned above as (D).

The tanned leather, skins or pelts produced according to the invention, as described above, may be further treated in conventional way, i.e. may be drained, dried and mechanically treated as usual for storage and/or shipment.

According to another preferred feature of the invention, the substrates are first tanned in one or two stages with (A), optionally subjected to complementary tanning with (F) or (F-II), and then are retanned with (F).

Retanning with (F) may be carried out after having rinsed, dried and optionally mechanically treated the tanned leather, skin or pelt e.g. in the dye-house.

The process of the invention may be carried out in a very economic an simple way, as a pickling and depickling may be omitted, and further the tanning itself may be carried out with a minimum quantity of water, and also a neutralisation - as otherwise conventionally carried out e.g. after metal tanning - is not necessary.

By the process of the invention there may be achieved metal-free tanned leathers, skins or pelts (in particular "wet white" metal-free leathers) of outstanding properties, in particular shrinkage temperatures, softness and consistency, e.g. firm grain texture, and with satisfactory fastnesses, especially where (A) or respectively (T) is employed for a main or full tanning. If no vegetable tanning agents at all are used or if only white to light yellowish vegetable tanning agents are also used, there may be achieved according to the invention "non-metal white tanned" leathers, skins and pelts, in particular non-metal "wet white" leathers, of high quality and very light own colour, i.e. nearly white. Where vegetable tanning agents of brownish to reddish colour are also used, the shade of the resulting tanned leathers, skins and pelts will be correspondingly slightly more brownish or reddish. Where (A) is employed for full tanning or for pre-tanning and main tanning and is followed by (F), in particular (F-II), for complementary tanning, further increased shrinkage temperatures T_{S} may be achieved.

The tanned leathers, skins and pelts produced as described are suitable for further treatment in conventional way, mainly by retanning and/or fatliquoring and optionally dyeing and/or finishing. Fatliquoring may be carried out with known fatliquoring agents. Retanning is preferably carried out with (F). By retanning them with (F) and fatliquoring there may be produced upon drying high quality crust leathers. For dyeing there may be employed known leather dyes (e.g. as defined and in particular listed in the "Colour Index edited by Society of Dyers and Colourists and American Association of Textile Chemists and Colorists") and there may be obtained dyeings of satisfactory properties, mainly colour penetration, colour yield and fastnesses. With those leathers, skins or pelts which are of very light own colour, i.e. are nearly white, as mentioned above, there may also be achieved dyeings of delicate shades (pastel shades) of very pleasant shade. Conventional leather finishing agents may also be employed for finishing if desired.

Therefore, another subject of the invention is the use of the tanned leather, skins or pelts produced according to the process as described above, also with all its preferred embodiments, for further processing by at least one further treatment selected from the group consisting of
(a) retanning with a further non-mineral tanning agent (F), which is different from the tanning agent (A),
(b) fat-liquoring,
(c) dyeing, and
(d) finishing.
preferably a and b and optionally c and/or d.

In the following examples the indicated percentages are by weight; the viscosities are Brookfield rotational viscosities determined according to standard ASTM D 2669.

### Examples

### Example 1

200 g of water are charged into a reactor with stirring and cooled to 1 - 3 °C. At this temperature 69 g of 2[(4-amino-3-phenyl sulphonic acid)sulphonyl]ethyl hydrogen sulphate of 72 % concentration are added and stirring is continued at 1 to 2 °C for 10 minutes. 35 g of an aqueous 30 % by weight of sodium hydroxide solution, the % by weight based on the weight of the solution, are added maintaining the temperature between 3 and 8 °C and the pH below 3. 100 g of ice and 25.4 g of cyanuric chloride are added and stirring is continued for 10 - 15 minutes. At this point 19 g of an aqueous 30 % by weight of sodium hydroxide solution, the % by weight based on the weight of the solution, are added over 7 - 8 hours, keeping the temperature below 10 °C and the pH in the range of 2.5 to 3.0, and stirring is continued for 30 minutes. There is obtained a white-yellowish dispersion (Composition 1) containing the compound of formula (1).

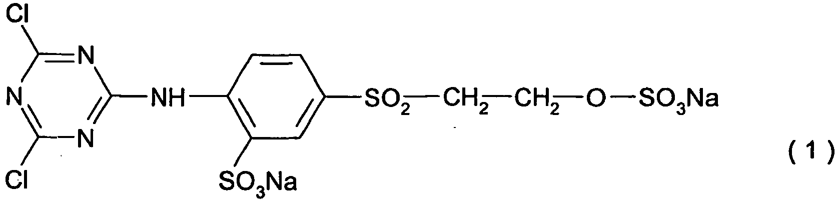

### Example 2

100 g of water are charged into a reactor with stirring and cooled to 1 - 3 °C. At this temperature 19.7 g of cyanuric chloride are added and stirring is continued at 1 to 2 °C for 10 minutes. 150 g of ice and and 30 g of 2[(4-aminophenyl)sulphonyl]ethyl hydrogen sulphate are added and stirring is continued for 10 - 15 minutes. 28.4 g of an aqueous 30 % by weight of sodium hydroxide solution, the % by weight based on the weight of the solution, are added over 3-4 hours maintaining the temperature below 10 °C and the pH at 2.5 - 3.0 Near the end of the sodium hydroxide solution the temperature is allowed to rise to 14 °C. After completion of the sodium hydroxide addition stirring is continued for 30 minutes

There is obtained a white-yellowish dispersion (Composition 2) containing the compound of formula (2).

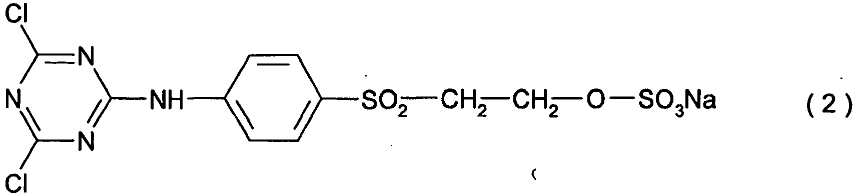

### Example 3

180 g of water are charged into a reactor at 20 - 25 °C, 39 g of 2[(4-amino-phenyl)sulphonyl]ethyl hydrogen sulphate are added and stirring is continued at 10 - 15 °C for 10 minutes. 30 g of tetrachloropyrimidine are added and stirring is continued for 10 - 15 minutes. At this point 37 g of an aqueous 30 % by weight of sodium hydroxide solution, the % by weight based on the weight of the solution, are added over 7 - 8 hours, keeping the temperature below 40 °C and the pH in the range of 6.0 to 7.0, and stirring is continued for 30 minutes. There is obtained a white-yellowish dispersion (Composition 3) containing the compounds of formulae (3a) and (3b).

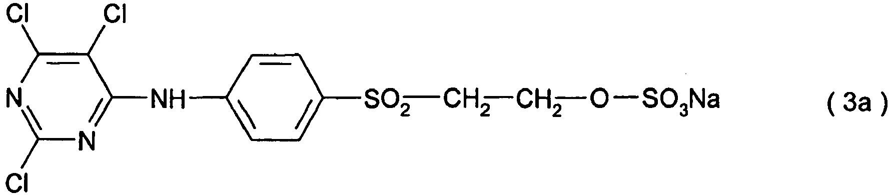

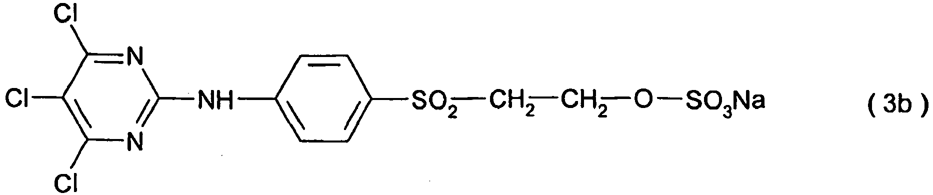

### Example 4

180 g of water are charged into a reactor at 20 - 25 °C, 69 g of 2[(4-amino-3-phenyl sulphonic acid)sulphonyl]ethyl hydrogen sulphate of 72 % concentration are added and stirring is continued at 10 - 15 °C for 10 minutes. 30 g of tetrachloropyrimidine are added and stirring is continued for 10 - 15 minutes. At this point 37 g of an aqueous 30 % by weight of sodium hydroxide solution, the % by weight based on the weight of the solution, are added over 7 - 8 hours, keeping the temperature below 40 °C and the pH in the range of 6.0 to 7.0, and stirring is continued for 30 minutes. There is obtained a white-yellowish dispersion (Composition 4) containing the compounds of formulae (4a) and (4b). 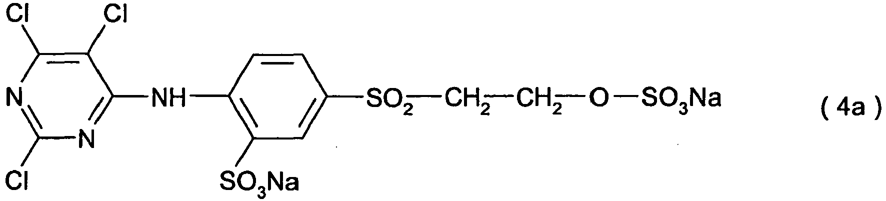 and

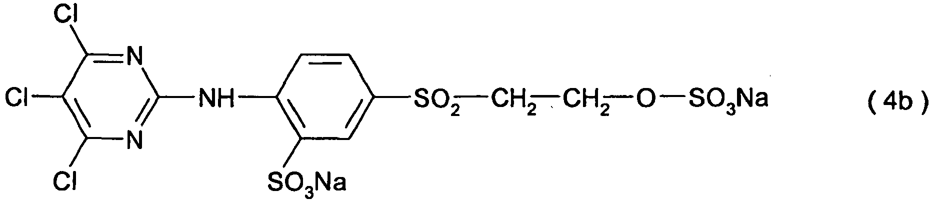

### Example 5

1,000 g of the Composition 1 obtained according to Example 1 are pre-set at 20 to 25 °C and 10.2 g of disodium hydrogen phosphate, 8.7 g of monosodium dihydrogen phosphate and 2.0 g of NIPACIDE^{®} BIT 20 (a commercial biocide based on 1,2-benzothiazolin-3-one of Clariant, Switzerland) are added with stirring. There is obtained a white-yellowish dispersion (Composition 5).

### Example 6

100 g of water are heated to 50 to 55 °C. At this temperature 4 g of hydroxyethylcellulose (having a viscosity of its 2 % solution of 5,500 mPa·s at 25 °C and a pH of 7.0) are added and stirring is continued for 1 hour. There are obtained 104 g of hydroxyethylcellulose solution.

1,000 g of Composition 5 obtained in Example 5 are pre-set at 20 to 25 °C and 103.8 g of the hydroxyethylcellulose solution is added with stirring. There is obtained a white-yellowish suspension (Composition 6) having a viscosity (Brookfield rotational viscosity, spindle nr. 3, 100 rpm) of 83 mPa·s at 20 °C.

### Example 7

Example 5 is repeated, with the difference that, instead of Composition 1 of Example 1, there is employed the same amount of Composition 2 of Example 2. There is obtained e white-yellowish dispersion (Composition 7)

### Example 8

Example 6 is repeated, with the difference that, instead of Composition 5 of Example 5, there is employed the same amount of Composition 7 of Example 7. There is obtained e white-yellowish suspension (Composition 8) having a viscosity of 2,010 mPa·s at 20 °C (Brookfield rotational viscosity, spindle nr. 3, 20 rpm).

### Examples 9 - 10

Example 5 is repeated, with the difference that, instead of Composition 1 of Example 1 there is employed the same amount of each of Compositions 3 or 4 of Examples 3 and 4. There are obtained white-yellowish dispersions (Compositions 9 and 10).

### Examples 9 - 10

Example 6 is repeated, with the difference that, instead of Composition 5 of Example 5 there is employed the same amount of each of Compositions 9 or 10 of Examples 9 and 10. There are obtained white-yellowish dispersions (Compositions 11 and 12).

In the following Application Examples the indicated percentages - if not otherwise indicated - refer in Application Examples Aa) and Ca) to the weight of the fleshed hide, in Application Examples B and D to the weight of the split hide, in Application Examples Ab) and Cb) to the weight of the bated hide, in Application Example E to the wet weight of the tanned leather. The shrinkage temperature T_{S} is determined according to standard method IUP 16 / ISO 3380-2006. Where a treatment is indicated to be carried out overnight, this is of 10 to 12 hours. If not otherwise indicated, pH is increased by addition of aqueous 10 % by weight of sodium formate solution, the % by weight being based on the weight of the solution or is lowered by addition of aqueous 10 % formic acid solution, the % by weight being based on the weight of the solution. The dyes are in commercial form blended with sodium chloride, with a dye content of around 60 %, "C.I." stands for "Colour Index".

### Application Example A

### a) Deliming and bating:

Bovine limed hide (French hide of the weight category 8 - 12 kg), fleshed and not split is charged into a drum with 200 % of water at 25 °C, 0.1 % of defatting agent (C₁₂₋₁₅ alkanol ethoxylated with 7 mols of ethylene oxide per mol of alkanol) and 0.2 % of an ammonium based deliming agent (ammonium chloride and ammonium sulphate) and drummed for 20 minutes. Then the bath is drained, a fresh bath of 50 % of water at 35 °C, 0.1 % of the above mentioned defatting agent and 0.5 % of the above mentioned ammonium based deliming agent is charged into the drum and drumming is continued for 15 minutes. A further 0.5 % of ammonium based deliming agent and 0.8 % of a mixture of 70 % boric acid and 30 % mixed organic acids (adipic, glutaric and succinic acids in even parts) are added and drumming is carried on for 90 minutes. The pH is 7.8 and the cross section of the hide is colourless to phenolphthalein. 0.6 % of Feliderm^{®} Bate PB1 p (a pancreas enzyme based bate of Clariant, Switzerland) is added and drumming is continued for 30 minutes and then the bath is drained. 300 % of water is added and drumming is carried on for 15 minutes at 35 °C then the bath is drained. T_{S} = 55 °C.

### b) Tanning:

A fresh bath of 50 % water at 20 °C is added and the pH is measured an adjusted to 8. 20 % of Composition 2 according to Example 2 is added and drumming is carried on for 60 minutes, then the bath is heated to 30 °C and drumming is continued overnight at 30 - 35 °C. Then the bath is drained off. 300 % of water at 20 °C is added and drumming is continued for 20 minutes and then the bath is drained and the leather discharged and horsed up. The shrinkage temperature T_{S} is 75 °C.

The bath is then adjusted to pH 8.5 - 10.0 with sodium carbonate and drummed for 2 hours. Then the pH is adjusted to 3.5 - 4.0 with formic acid and drumming is carried on for 7-8 hours at 40 - 45 °C
Then the bath is drained off. T_{S} = 77 °C

### c) Complementary tanning

300 % of water is added and drumming is continued for 20 minutes. 1 % of syntan based on sulphomethylated dihydroxydiphenylsulphone reacted with formaldehyde is added for complementary tanning and drumming is continued for 120 minutes and then the bath is drained. 300 % of water at 20 °C is added and drumming is continued for 30 minutes and then the bath is drained off, the leather is discharged and horsed-up,

The so obtained leather is then sammied, split and shaved to 1.0 to 1.1 mm.

### Application Example B

### a) Deliming and bating:

Bovine limed hide (French hide of the weight category 8-12 kg), fleshed and split to a thickness of 2.4 - 2.5 mm, is charged into a drum with 200 % of water at 25 °C, 0.1 % of defatting agent (C₁₂₋₁₅ alkanol ethoxylated with 7 mols of ethylene oxide per mol of alkanol) and 0.2 % of an ammonium based deliming agent (ammonium chloride and ammonium sulphate) and drummed for 20 minutes.
Then the bath is drained, a fresh bath of 50 % of water at 35 °C, 0.1 % of the above mentioned defatting agent and 0.5 % of the above mentioned ammonium based deliming agent is charged into the drum and drumming is continued for 15 minutes. A further 0.5 % of ammonium based deliming agent and 0.8 % of a mixture of 70 % boric acid and 30 % mixed organic acids (adipic, glutaric and succinic acids in even parts) are added and drumming is carried on for 90 minutes. The pH is 7.8 and the cross section of the hide is colourless to phenolphthalein. 0.6 % of Feliderm^{®} Bate PB1 p (a pancreas enzyme based bate of Clariant, Switzerland) is added and drumming is continued for 30 minutes and then the bath is drained. 300 % of water is added and drumming is carried on for 15 minutes at 35 °C then the bath is drained. T_{S} = 54 °C.

### b) Tanning:

A fresh bath of 50 % water at 20 °C is added; the pH is 8. 20 % of Composition 2 according to Example 2 is added and drumming is carried on for 60 minutes, then the bath is heated during 120 minutes to 30 °C and drumming is continued overnight at 30 - 35 °C. The bath is then adjusted to pH 9.0 - 10.0 with sodium carbonate and drummed for two hours. Thereafter the pH is adjusted to 3.5 with formic acid and drumming is continued for 7 - 8 hours at 40 - 45 °C, then the bath is drained off.
The shrinkage temperature T_{S} is 76 °C.

### c) Complementary tanning:

300 % of water is added and drumming is carried on for 15 minutes. Then 2 % of Tara (commercial vegetable tanning agent, which is an aqueous composition of 50 % by weight concentration, based on the weight of the composition, of an extract of the pods of Caesalpinia Spinosa) is added and drumming is continued for 3 hours at 35 °C. Then the bath is drained off. 300 % of water at 20 °C is added and drumming is continued for 30 minutes. Then the bath is drained off, the leather is discharged and horsed up. The so obtained leather is then sammied, split and shaved to 1.0 to 1.1 mm.

### Application Example C

### a) Deliming and bating:

Bovine limed hide (Spanish bull hide of the weight category 30 kg), fleshed and not split is charged into a drum with 200 % of water at 25 °C, 0.1 % of defatting agent (C₁₂₋₁₅ alkanol ethoxylated with 7 mols of ethylene oxide per mol of alkanol) and 0.2 % of an ammonium based deliming agent (ammonium chloride and ammonium sulphate) and drummed for 20 minutes. Then the bath is drained, a fresh bath of 50 % of water at 35 °C, 0.1 % of the above mentioned defatting agent and 0.5 % of the above mentioned ammonium based deliming agent is charged into the drum and drumming is continued for 15 minutes. A further 0.5 % of ammonium based deliming agent and 0.8 % of a mixture of 70 % boric acid and 30 % mixed organic acids (adipic, glutaric and succinic acids in even parts) are added and drumming is carried on for 90 minutes. The pH is 7.8 and the cross section of the hide is colourless to phenolphthalein indicator solution. 0.6 % of Feliderm^{®} Bate PB1 p is added and drumming is continued for 30 minutes and then the bath is drained. 300 % of water is added and drumming is carried on for 15 minutes at 35 °C then the bath is drained. T_{S} = 55 °C.

### b) Tanning:

A fresh bath of 50 % water at 20 °C is added. The pH is measured and adjusted to 8. 20 % of Composition 3 according to Example 3 is added and drumming is carried on for 60 minutes, then the bath is heated to 30 °C and drumming is continued overnight at 30 - 35 °C. Then the bath is drained off. 300 % of water at 20 °C is added and drumming is continued for 20 minutes.

If desired 0.2 % of Preventol^{®} WB is added to the last 300 % of water. Then the bath is drained off, the leather is discharged and horsed up. The shrinkage temperature T_{S} is 68 °C.

### c) Complementary tanning:

300 % of water is added and drumming is continued for 20 minutes. 1 % of syntan based on sulphomethylated dihydroxydiphenylsulphone reacted with formaldehyde is added for complementary tanning and drumming is continued for 120 minutes and then the bath is drained. 300 % of water at 20 °C is added and drumming is continued for 30 minutes and then the bath is drained off, the leather is discharged and horsed-up. T_{S} = 70 °C
The so obtained leather is then sammied and shaved to 2.0 to 2.2.1 mm.

### Application Example D

### a) Deliming and bating:

Bovine limed hide (Spanish bull hide of the weight category 30 kg), fleshed and not split is charged into a drum with 200 % of water at 25 °C, 0.1 % of defatting agent (C₁₂₋₁₅ alkanol ethoxylated with 7 mols of ethylene oxide per mol of alkanol) and 0.2 % of an ammonium based deliming agent (ammonium chloride and ammonium sulphate) and drummed for 20 minutes. Then the bath is drained, a fresh bath of 50 % of water at 35 °C, 0.1 % of the above mentioned defatting agent and 0.5 % of the above mentioned ammonium based deliming agent is charged into the drum and drumming is continued for 15 minutes. A further 0.5 % of ammonium based deliming agent and 0.8 % of a mixture of 70 % boric acid and 30 % mixed organic acids (adipic, glutaric and succinic acids in even parts) are added and drumming is carried on for 90 minutes. The pH is 7.8 and the cross section of the hide is colourless to phenolphthalein indicator solution. 0.6 % of Feliderm^{®} Bate PB1 p is added and drumming is continued for 30 minutes and then the bath is drained. 300 % of water is added and drumming is carried on for 15 minutes at 35 °C then the bath is drained. T_{S} = 55 °C.

### b) Tanning:

A fresh bath of 50 % water at 20 °C is added. The pH is measured and adjusted to 8. 20 % of Composition 2 according to Example 2 is added and drumming is carried on for 60 minutes, then the bath is heated to 30 °C, 1 % of polyacrylates resin is added and drumming is continued overnight at 30 - 35 °C. Then the bath is drained off. 300 % of water at 20 °C is added and drumming is continued for 20 minutes.

If desired 0.2 % of Preventol^{®} WB is added to the last 300 % of water.

The bath is then adjusted to pH9.0-10.0 with sodium carbonate and drummed for 2 hours. Thereafter the pH is adjusted to 4.0 with formic acid and drumming is continued for 7 - 8 hours at 40 - 45 °C.

Then the bath is drained off, the leather is discharged and horsed up. The shrinkage temperature T_{S} is 77 °C.

### Application Example E

The leather obtained in Application Example C is retanned, fatliquored and dyed as follows:
The leather is charged into the drum, 200 % of water at 25 °C and then 0.3 % of defatting agent (C₁₂₋₁₅ alkanol ethoxylated with 7 mols of ethylene oxide per mol of alkanol) are added, the drum is switched on and drumming is carried on for 20 minutes. 0.5 % acetic acid, diluted 1:10 is added and drumming is continued for 20 minutes. The pH is 4.5 and the cross section of the leather turns green by testing with Bromocresol Green indicator. The bath is drained off. 100 % of water at 25 °C and then 1.6 % of fatliquoring agent (oxy-sulphited fish oil) are added and drumming is continued for 20 minutes. 5 % of a retanning syntan based on sulphomethylated dihydroxydiphenylsulphone reacted with formaldehyde and 5 % of a phenolic syntan (reaction product of sulphonated phenol with formaldehyde and urea) are added and drumming is carried on for 2 hours. The bath is allowed to dwell overnight with intermittent drumming for 5 minutes every hour, then 0.5 % of formic acid diluted 1:10 is added and drumming is carried on for 20 minutes, then the bath is drained off and the leather is washed with 200 % of water. The bath is drained off. 100 % of water at 50 °C is added followed by 5 % of fatliquoring agents (3.5 % alkylsulphosuccinate and 1.5 % oxy-sulphited fish oil) and drumming is continued for 1 hour. After addition of 0.5 % of formic acid drumming is carried on for 20 minutes and then the bath is drained off. The leather is rinsed for 5 minutes with 200 % of water at 20 °C. The bath is then drained off. 50 % of water at 20 °C and 5 % of the black dye C.I. Acid Black 210 is added and drumming is continued for 1 hour, then 200 % of water at 50 °C and 1 % of formic acid are added and drumming is continued for 10 minutes, then a further 1 % of formic acid is added and drumming is carried on for 20 minutes, then the bath is drained off. 200 % of water at 20 °C and 1.5 % of a cationic surfactant 2-(8-heptadecenyl)-4,5-dihydro-1,3-bis(2-hydroxyethyl)-1H-imidazolium chloride are added and drumming is carried on for 15 minutes, then the bath is drained off and the leather is discharged. After 24 hours it is set out, vacuum dried at 60 °C during 2 minutes, dried hanging and staked. There is obtained a black dyed leather of satisfactory properties.

By employing 2 % the brown dye C.I. Acid Brown 237 instead of the 5 % of the black dye C.I. Acid Black 210, there is obtained a brown dyed leather of satisfactory properties.

Analogously as the leather from Application Example C also the leathers obtained according to each of Application Examples A, B and D are retanned, fatliquored and dyed according to the procedure described in Application Example E.

In the above Application Examples there are obtained leathers of satisfactory commercial grade in particular with satisfactory grain tightness, texture consistency (e.g. as resulting from some typical properties such as tensile strength, tear load and stitch tear resistance), softness, fastnesses and general appearance. In the dyeing examples there are further obtained dyed leathers of satisfactory properties in particular shade, dye penetration and colour yield, and fastnesses of the dyeing.

## Claims

1. A process for the production of tanned leather, skins or pelts by non-metal tanning, comprising the step of tanning a bated hide, skin or pelt with a tanning agent (A), the tanning agent (A) being at least one compound of formula (I),
R-NH-Y-SO₂-Z (I),
wherein
R signifies a radical of formula 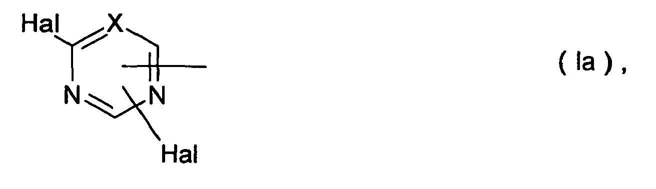 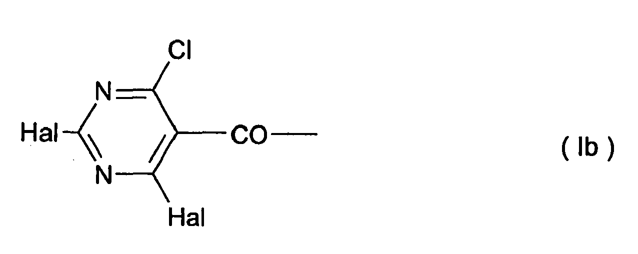 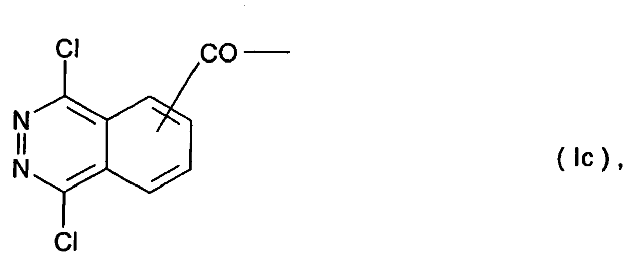 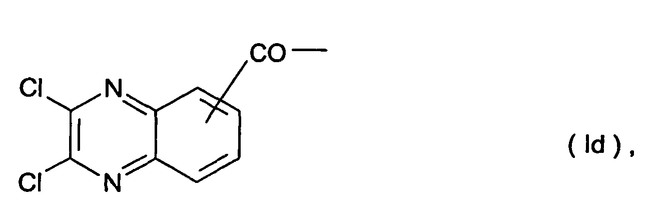 or 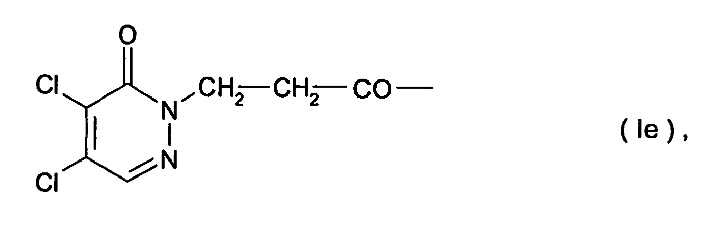 wherein
Hal signifies fluorine or chlorine,
X signifies N or CR1,
Y signifies an aliphatic, araliphatic or aromatic hydrocarbonic bridge which may optionally be interrupted by a heteroatom or heteroatomic group,
Z signifies vinyl, β-chloroethyl, β-phosphatoethyl or β-sulphatoethyl
R1 signifies hydrogen or Cl,
in a tanning bath, the tanning bath having a pH of from 6 to 10 at the beginning of tanning step.

2. A tanning process according to Claim 1, wherein the bated hide or skin or pelt is subjected to tanning with (A) without previous pickling.

3. A process according to Claim 1, wherein a bated and pickled hide, skin or pelt is depickled to a pH in the range of 6 to 10 before tanning with (A).

4. A process according to any one of Claims 1 to 3, wherein the tanning agent (A) is employed in the form of an aqueous composition (T) free of metal compounds of tanning activity.

5. A process according to Claim 4, wherein the composition (T) is a composition (T1), the composition (T1) further comprising a surfactant (B) and/or a buffer (C₁) to keep an acidic to neutral pH.

6. A process according to Claim 5, wherein the composition (T1) further comprises an agent (D) to protect against the damaging action of microorganisms and/or a polysaccharide-based thickener (E).

7. A process according to Claim 6, wherein aqueous composition (T1) comprises (E).

8. A process according to any one of Claims 1 to 7, wherein the tanning bath comprises a buffer (C2) to achieve a nearly neutral to basic pH at the beginning of the tanning step.

9. A process according to any one of Claims 1 to 8 for the production of non-metal tanned leather, skin or pelt, wherein the tanning step with the tanning agent (A) is a pre-tanning, a main tanning or a full tanning or a pretanning and a main tanning.

10. A process according to Claim 9, wherein a non-mineral tanning agent (F), which is different from the tanning agent (A) as defined in claim 1, is used before, after or together with tanning agent (A) in pre-tanning, in main tanning or in full tanning, or in combination with tanning agent (A) in full tanning.

11. A process according to Claim-10 wherein the non-mineral tanning agent (F) is selected from the group consisting of
(F1) a vegetable tanning agent,
(F2) a syntan,
(F3) a synthetic, semisynthetic or natural resin or polymer,
(F4) a tanning natural oil or modified oil,
(F5) a 4,6-dichloro-1,3,5-triazin-2-yl-aminobenzene-mono- or di-sulphonic acid salt.
(F6) a tanning oxazolidine,
and
mixtures of two or more thereof.

12. A process according to Claim 9, wherein the substrates are tanned with tanning agent (A) in a main or full tanning and then are subjected to a complementary tanning with a non-mineral tanning agent (F) as defined in claim 10 or 11.

13. A process according to claims 10, 11, or 12 wherein the non-mineral tanning agent (F) is employed in a smaller amount compared with the amount of the tanning agent (A).

14. Use of the tanning composition (T1) as defined in claims 5, 6 or 7 as tanning agent (A) in a tanning process as defined in any one of Claims 1 to 13.

15. Use of the tanned leather, skins or pelts produced according to a process as defined in anyone of Claims 1 to 13 for further processing by at least one further treatment selected from the group consisting of
(a) retanning with a non-mineral tanning agent (F) as defined in claims 10 or 11, which is different from the tanning agent (A) as defined in claim 1,
(b) fat-liquoring,
(c) dyeing, and
(d) finishing.

16. Use of the tanned leather, skins or pelts produced according to a process as defined in anyone of Claims 1 to 13 for further processing by a further treatment comprising retanning with a non-mineral tanning agent (F) as defined in claims 10 or 11.

17. Use of the tanned leather, skins or pelts produced according to a process as defined in anyone of Claims 1 to 13 for further processing by a further treatment comprising retanning with non-mineral tanning agent (F) as defined in claims 10 or 11, fat-liquoring and optionally dyeing and/or finishing.

18. Tanned leather, skins or pelts obtainable by a process according to any one of Claims 1 to 13.

## Patentansprüche

1. Verfahren zur Herstellung von gegerbtem Leder, Häuten oder Pelzen durch nicht-metallisches Gerben, umfassend den Schritt des Gerbens eines abgenommenen Fells, einer Haut oder eines Pelzes mit einem Gerbmittel (A), wobei das Gerbmittel (A) mindestens eine Verbindung der Formel (I).
R-NH-Y-SO2-Z (I)
ist, wobei
R ein Radikal der Formel 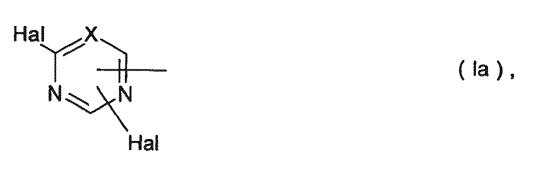 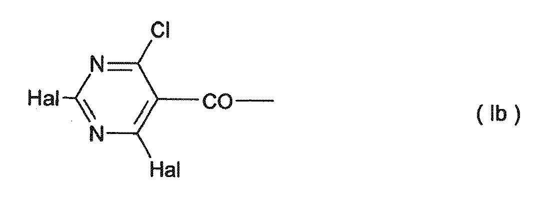 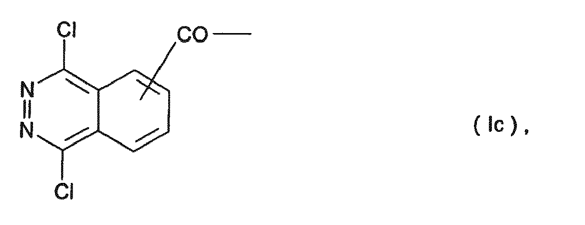 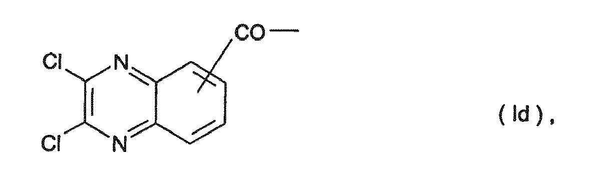 oder 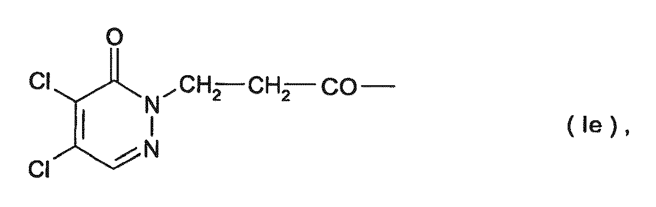 bezeichnet, wobei
Hal Fluor oder Chlor bezeichnet,
X N oder CR1 bezeichnet,
Y eine aliphatische, araliphatische oder aromatische Kohlenwasserstoffbrücke bezeichnet, die optional durch ein Heteroatom oder eine heteroatomische Gruppe unterbrochen sein kann,
Z Vinyl, β-Chlorethyl, β-Phosphatethyl oder β-Sulfatethyl bezeichnet,
R1 Wasserstoff oder CI bezeichnet,
in einem Gerbbad, wobei das Gerbbad zu Beginn des Gerbschritts einen pH-Wert von 6 bis 10 hat.

2. Gerbverfahren nach Anspruch 1, wobei das abgenommene Fell, die Haut oder der Pelz der Gerbung mit (A) ohne vorhergehendes Beizen unterzogen wird.

3. Verfahren nach Anspruch 1, wobei ein abgenommenes und gebeiztes Fell, eine Haut oder ein Pelz vor dem Gerben mit (A) auf einen pH-Wert im Bereich von 6 bis 10 entbeizt wird.

4. Verfahren nach einem der Ansprüche 1 bis 3, wobei das Gerbmittel (A) in Form einer wässrigen Zusammensetzung (T) frei von Metallverbindungen mit Gerbaktivität angewendet wird.

5. Verfahren nach Anspruch 4, wobei die Zusammensetzung (T) eine Zusammensetzung (T1) ist, wobei die Zusammensetzung (T1) ferner ein Tensid (B) und/oder einen Puffer (C₁) umfasst, um einen sauren bis neutralen pH-Wert aufrecht zu erhalten.

6. Verfahren nach Anspruch 5, wobei die Zusammensetzung (T1) ferner ein Mittel (D) als Schutz gegen die schädigende Wirkung von Mikroorganismen und/oder ein Eindickungsmittel (E) auf Polysaccharidbasis umfasst.

7. Verfahren nach Anspruch 6, wobei die wässrige Zusammensetzung (T1) (E) umfasst.

8. Verfahren nach einem der Ansprüche 1 bis 7, wobei das Gerbbad einen Puffer (C2) umfasst, um zu Beginn des Gerbschritts einen fast neutralen bis basischen pH-Wert zu erhalten.

9. Verfahren nach einem der Ansprüche 1 bis 8 zur Herstellung von nicht-metallisch gegerbtem Leder, Haut oder Pelz, wobei der Gerbschritt mit dem Gerbmittel (A) eine Vorgerbung, eine Hauptgerbung oder eine Vollgerbung oder eine Vorgerbung und eine Hauptgerbung ist.

10. Verfahren nach Anspruch 9, wobei ein nicht-mineralisches Gerbmittel (F), das sich von dem Gerbmittel (A), wie in Anspruch 1 definiert, unterscheidet, vor, nach oder zusammen mit dem Gerbmittel (A) bei der Vorgerbung, der Hauptgerbung oder der Vollgerbung oder in Kombination mit dem Gerbmittel (A) bei der Vollgerbung verwendet wird.

11. Verfahren nach Anspruch 10, wobei das nicht-mineralische Gerbmittel (F) ausgewählt ist aus der Gruppe bestehend aus
(F1) einem pflanzlichen Gerbmittel,
(F2) einem Syntan,
(F3) einem synthetischen, halbsynthetischen oder natürlichen Harz oder Polymer,
(F4) einem natürlichen oder modifizierten Grerböl,
(F5) einem 4,6-Dichlor-1,3,5-triazin-2-yl-aminobenzen-mono- oder disulfonsäuresalz,
(F6) einem Gerboxazolidin,
und
Gemischen von zwei oder mehr davon.

12. Verfahren nach Anspruch 9, wobei die Substrate mit Gerbmittel (A) in einer Haupt- oder Vollgerbung gegerbt werden und dann einer ergänzenden Gerbung mit einem nicht-mineralischen Gerbmittel (F) wie in Anspruch 10 oder 11 definiert unterzogen werden.

13. Verfahren nach den Ansprüchen 10, 11 oder 12, wobei das nicht-mineralische Gerbmittel (F) in einer geringeren Menge verglichen mit der Menge des Gerbmittels (A) angewendet wird.

14. Verwendung der Gerbzusammensetzung (T1), wie in den Ansprüchen 5, 6 oder 7 definiert, als Gerbmittel (A) in einem Gerbverfahren, wie in einem der Ansprüche 1 bis 13 definiert.

15. Verwendung des gegerbten Leders, der Häute oder Pelze nach einem Verfahren, wie in einem der Ansprüche 1 bis 13 definiert, zur weiteren Verarbeitung durch mindestens eine weitere Behandlung, ausgewählt aus der Gruppe bestehend aus
(a) erneutem Gerben mit einem nicht-mineralischen Gerbmittel (F) wie in den Ansprüchen 10 oder 11 definiert, das sich von dem Gerbmittel (A), wie in Anspruch 1 definiert, unterscheidet,
(b) Fetten,
(c) Färben und
(d) Veredeln.

16. Verwendung des gegerbten Leders, der Häute oder Pelze, hergestellt nach einem Verfahren, wie in einem der Ansprüche 1 bis 13 definiert, zur weiteren Verarbeitung durch eine weitere Behandlung, umfassend das erneute Gerben mit einem nicht-mineralischen Gerbmittel (F), wie in den Ansprüchen 10 oder 11 definiert.

17. Verwendung des gegerbten Leders, der Häute oder Pelze, hergestellt nach einem Verfahren, wie in einem der Ansprüche 1 bis 13 definiert, zur weiteren Verarbeitung durch eine weitere Behandlung umfassend das erneute Gerben mit einem nicht-mineralischen Gerbmittel (F), wie in den Ansprüchen 10 oder 11 definiert, Fetten und optional Färben und/oder Veredeln.

18. Gegerbtes Leder, Häute oder Pelze, erhältlich durch ein Verfahren nach einem der Ansprüche 1 bis 13.

## Revendications

1. Procédé de production de cuir, de peaux, ou de fourrures tannés par un tannage non métallique, comprenant l'étape consistant à tanner un masque, une peau ou une fourrure retenant avec un agent de tannage (A), l'agent de tannage (A) étant au moins un composé de formule (I)
R-NH-Y-SO₂-Z (I)
où
R signifie un radical de formule 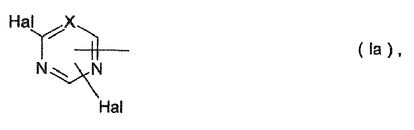 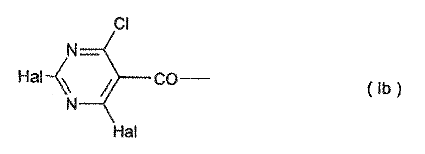 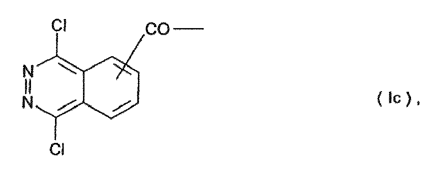 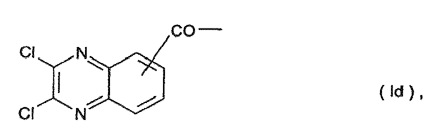 ou 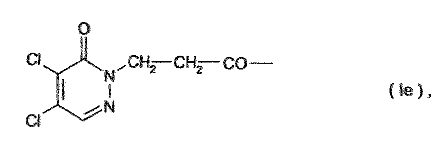 où
Hal signifie du fluor ou du chlore,
X signifie N ou CR1,
Y signifie un pont hydrocarbonique aliphatique, araliphatique ou aromatique qui peut facultativement être interrompu par un hétéroatome ou un groupe hétéroatomique,
Z signifie vinyle, β-chloroéthyle, β-phosphatoèthyle ou β-sulfatoéthyle,
R1 signifie hydrogène ou Cl
dans un bain de tannage, le bain de tannage ayant un pH de 6 à 10 au début de l'étape de tannage.

2. Procédé selon la revendication 1, dans lequel le masque, la peau ou la fourrure retenant est soumis(e) à un tannage avec (A) sans décapage préalable.

3. Procédé selon la revendication 1, dans lequel un masque, une peau ou une fourrure retenant et picklé(e) est dépicklé(e) à un pH dans la plage de 6 à 10 avant tannage avec (A).

4. Procédé selon l'une quelconque des revendications 1 à 3, dans lequel l'agent de tannage (A) est utilisé sous la forme d'une composition aqueuse (T) sans composé métallique ayant une activité de tannage.

5. Procédé selon la revendication 4, dans lequel la composition (T) est une composition (T1), la composition (T1) comprenant de plus un tensioactif (B) et/ou un tampon (C₁) pour maintenir un pH acide à neutre.

6. Procédé selon la revendication 5, dans lequel la composition (T1) comprend de plus un agent (D) de protection contre l'action de détérioration de microorganismes et/ou un épaississeur à base de polysaccharide (E).

7. Procédé selon la revendication 6, dans lequel la composition aqueuse (T1) comprend (E).

8. Procédé selon l'une quelconque des revendications 1 à 7, dans lequel le bain de tannage comprend un tampon (C2) pour obtenir un pH pratiquement neutre à basique au début de l'étape de tannage.

9. Procédé selon l'une quelconque des revendications 1 à 8, pour la production d'un cuir, d'une peau ou d'une fourrure tanné(e) sans métal, dans lequel l'étape de tannage avec l'agent de tannage (A) est un pré-tannage, un tannage principal ou un tannage complet, ou un pré-tannage et un tannage principal.

10. Procédé selon la revendication 9, dans lequel un agent de tannage non minéral (F), qui est différent de l'agent de tannage (A) tel que défini dans la revendication 1, est utilisé avant, après ou avec l'agent de tannage (A) dans un pré-tannage, un tannage principal ou un tannage complet, ou en combinaison avec l'agent de tannage (A) dans un tannage complet.

11. Procédé selon la revendication 10, dans lequel l'agent de tannage non minéral (F) est sélectionné parmi le groupe comprenant :
(F1) : un agent de tannage végétal,
(F2) : un syntan,
(F3) : une résine ou un polymère synthétique, semi-synthétique ou naturel,
(F4) : une huile de tannage naturelle ou une huile de tannage modifiée,
(F5) : un sel d'acide 4,6-dichloro-1,3,5-triazin-2-yl-aminobenzène- mono ou di-sulfonique
(F6) : une oxazolidine de tannage
et
des mélanges de deux ou plus de deux de ceux-ci.

12. Procédé selon la revendication 9, dans lequel les substrats sont tannés avec l'agent de tannage (A) dans un tannage principal ou complet, et sont ensuite soumis à un tannage complémentaire avec un agent de tannage non minéral (F), tel que défini dans les revendications 10 ou 11

13. Procédé selon les revendications 10, 11 ou 12, dans lequel l'agent de tannage non minéral (F) est utilisé dans une quantité plus petite par comparaison à la quantité de l'agent de tannage (A).

14. Utilisation de la composition de tannage (T1) telle que définie dans les revendications 5, 6 ou 7 comme agent de tannage (A) dans un procédé de tannage tel que défini dans l'une quelconque des revendications 1 à 13.

15. Utilisation de cuir, de peaux ou de fourrures tanné(e)s selon un procédé tel que défini dans l'une quelconque des revendications 1 à 13, pour traiter par au moins un traitement supplémentaire sélectionné parmi le groupe consistant à :
(a) re-tanner avec un agent de tannage non minéral (F) tel que défini dans les revendications 10 ou 11, qui est différent de l'agent de tannage (A) tel que défini dans la revendication 1,
(b) graisser,
(c) sécher, et
(d) finir.

16. Utilisation de cuir, de peaux ou de fourrures tanné(e)s selon un procédé tel que défini dans l'une quelconque des revendications 1 à 13 pour traiter de plus par un autre traitement consistant à re-tanner avec un agent de tannage non minéral (F) tel que défini dans les revendications 10 ou 11.

17. Utilisation de cuir, de peaux ou de fourrures tanné(e)s selon un procédé tel que défini dans l'une quelconque des revendications 1 à 13 pour traiter de plus par un autre traitement consistant à re-tanner avec un agent de tannage non minéral (F) tel que défini dans les revendications 10 ou 11, graisser et facultativement sécher et/ou finir.

18. Cuir, peaux ou fourrures tanné(e)s pouvant être obtenu(e)s par un procédé selon l'une quelconque des revendications 1 à 13
